# EUROPEAN PATENT APPLICATION

(11) **EP 4 566 660 A1**
(43) Date of publication of application: **11.06.2025**
(21) Application number: 24214863.3
(22) Date of filing: 22.11.2024
(51) Int. Cl.: A61N 2/00, A61H 9/00, A61N 1/40, A61N 2/02

(54) **VERSATILE MULTIFUNCTIONAL DEVICE FOR PRESSOTHERAPY AND/OR MAGNETOTHERAPY, AS WELL AS A COMPUTER PROGRAM FOR THE OPERATION THEREOF**

(30) Priority: 04.12.2023 IT 202300025857
(71) Applicant: Amel Medical Division S.r.l., 36016 Thiene (VI) (IT)
(72) Inventor: POLI, Luigi, 35010 San Pietro In Gu (PD) (IT)
(74) Representative: Autuori & Partners S.R.L.

(57) **Abstract**

A versatile multifunctional device for pressotherapy and/or magnetotherapy, which can be used by a user to treat at least one or more areas of the body, comprising a shell (**100**) defining an inner compartment (**110**) which includes a logic control unit (**50**), power supply means (**10**), pneumatic means (**30**). The device further comprises a support (**40**) which can be anchored to the user's area to be treated, comprising a first portion (**41**) which includes diffuser means (**21**) for magnetotherapy and a second portion (**42**) separated from the first and comprising at least one air chamber (**31**) for pressotherapy fluidically connected to the pneumatic means (**30**). The control means (**50**) alternatively or simultaneously actuates the diffuser means (**21**) and the pneumatic means (**30**) so as to treat the at least one area of the user's body.

## Description

### Field of the invention

The present invention generally relates to the technical field of devices and it particularly relates to a versatile multifunctional device for pressotherapy and/or magnetotherapy for treating at least one area of the body of a user, as well as a computer programme for the operation thereof.

### State of the Art

Pressotherapy devices which comprise an electronic equipment connected to applicators which can be anchored to the user's anatomical area to be treated are known.

Typically, the areas to be treated are limbs of the user and the abdominal area.

Therefore, the applicators are in the form of legs, cuffs or abdominal bands which can be worn by the users.

The applicators typically comprise several air chambers that are consecutive and not placed in communication with each other which are inflated sequentially using a compressor.

Such air chambers exert a graduated and rhythmic compression to the area to be treated so as to facilitate blood circulation, improve lymphatic drainage and reduce fluid retention.

In addition, devices for pressotherapy integrated with infrared ray heat generator devices or cryotherapy devices for a respectively warm or cold effect, are known.

On the other hand, devices for magnetotherapy which use magnetic fields induced by the electric current are known. The latter passes through a solenoid contained in a diffuser for generating a low frequency field, between 5 Hz and 500 Hz, or a conductive grating contained in a diffuser, typically a natural fibre fabric with fibres integrated in copper, to generate a high frequency field, between 10 MHz and 900 MHz.

Specifically, there are known devices consisting of a machine which controls one or more diffusers to be applied at the area to be treated, adjusting the frequency and/or the force of the magnetic field depending on the therapy programme selected and on the desired effect.

However, all prior art devices are susceptible to improvements.

### Summary of the invention

An object of the present invention is to at least partly overcome the aforementioned drawbacks, by providing a device for treating at least one area of the body of the user, that is ultra versatile.

Another object of the invention is to provide a device for treating at least one area of the body of a user, that is particularly powerful.

A further object of the present invention is to at least partially overcome the aforementioned drawbacks, by providing a device for treating at least one area of the body of a user, that is easy to use and cost-effective.

Another object of the invention is to provide a device for treating at least one area of the body of a user, that is small in size.

These and other objects that will be more apparent hereinafter, are attained by a versatile multifunctional device and/or a computer programme as described, illustrated and/or claimed herein.

The dependent claims describe advantageous embodiments of the invention.

### Brief description of the drawings

Further characteristics and advantages of the invention will be more apparent in the light of the detailed description of some preferred but non-exclusive embodiments of a multifunction device, shown by way of non-limiting example with reference to the attached drawings, wherein:
**FIGS. 1A** and **1B** are schematic views of the inner part of the device **1**;
**FIG. 2** is an axonometric schematic view of the device **1**;
**FIG. 3** is a schematic view of the system **2**, wherein the support **40** is in a possible embodiment.

### Detailed description of some preferred embodiments

With reference to the figures mentioned above, herein described is a device 1 which may be a medical device in accordance with Directive 93/42/EC and/or regulation (EU) 2017/745 and/or legislation in force at the time and in the state of interest or an aesthetic device or generally a device for the well-being of a user.

Furthermore, such device may be used for domestic or professional purposes.

The present invention may include various parts and/or similar or identical elements. Unless otherwise specified, similar or identical parts and/or elements will be indicated using a single reference number, it being clear that the described technical characteristics are common to all similar or identical parts and/or elements.

Generally, the multifunction device **1** may be suitable for pressotherapy, magnetotherapy or pressotherapy and magnetotherapy combined, for treating one or more areas of the body of a user.

Firstly, the device **1** may include at least one shell **100,** preferably one, with at least one inner compartment **110,** preferably one, which may include the components required for the operation thereof.

Preferably, the compartment **110** may include power supply means **10,** pneumatic means **30** and a logic control unit **50.**

It is clear that several logic control units **50** may be provided for without departing from the scope of protection of the attached claims.

In a preferred but non-exclusive embodiment, the power supply means **10** may be rechargeable.

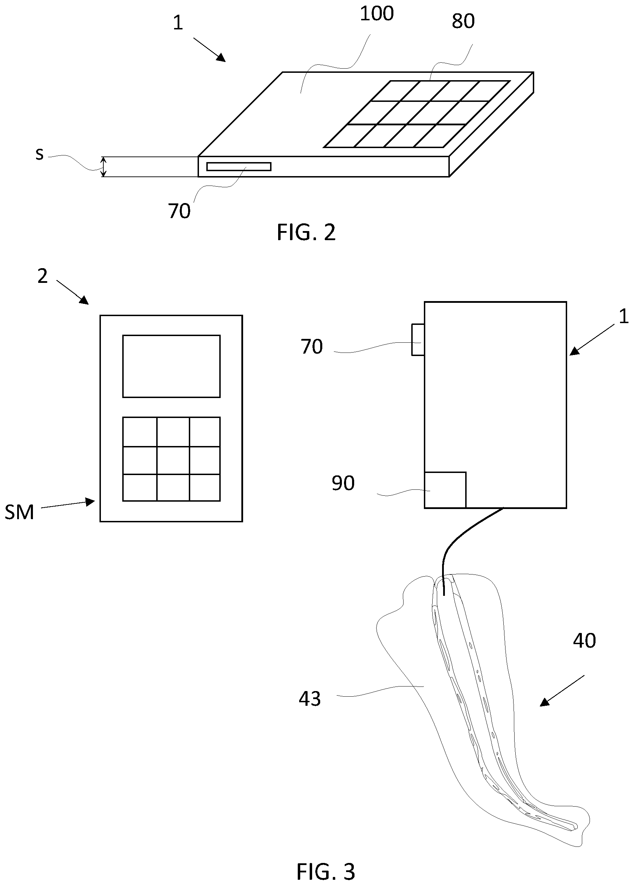

The device **1** may therefore be portable and in this case the shell **100** may be for example a compact palmtop device with a body defining the shell **100,** as particularly shown in **FIG. 2****.**

Should the power supply means **10** be rechargeable, they may be charged by connecting with the power mains and they may power-supply the device 1 both when charging and even when they will be disconnected from the power mains.

On the other hand, the power supply means **10** may include a specific circuitry which can be connected to the power mains.

In this case, the device **1** may be not portable and it may operate solely when the power supply means **10** will be connected the power mains.

Suitably, both the power supply means **10,** and the pneumatic means **30** may be connected to a logic control unit **50.**

On the other hand , the device **1** may include one or more supports **40** which can be anchored to the user's area to be treated.

For example, the supports **40** may be in the form of a leg, a cuff, an abdominal band or a vertebral support mattress .

It is clear that, the supports **40** may therefore fully surround the area to be treated or have a portion in contact with the area to be treated.

For example, a leg or a cuff may fully surround the limb, an abdominal band may fully surround the abdomen, while a mattress may be in contact with the back and the vertebral region.

It is therefore clear that the device **1** may be provided with one or more supports **40** of several types or of a single type without departing from the scope of protection of the attached claims.

For the sake of description simplicity, reference will be made to a single support. However, it is clear that the description outlined below may identically refer to each support, of any type, provided that it can be anchored to an area of the user to be treated.

Preferably, the support **40** may comprise a portion **41** with diffuser means **21** for magnetotherapy, connected to a logic control unit **50.**

The support **40** may comprise a portion **42** with one or more air chambers **31** for pressotherapy, fluidically connected to the pneumatic means **30.**

Preferably, the portion **41** and the portion **42** may be separated from each other.

As a matter of fact, in a preferred but non-exclusive embodiment, the support **40** may include a casing **43** with an internal chamber **430** and an internal separator **44.**

For example, the casing **43** may be made of technical fabric or natural fibre and it may include cut out made of the same material or a different material, defining the separator **44.**

Per se, the separator **44** may be fixed to the inner walls of the casing **43** or there may be provided for a single cloth of material, folded in a per se known manner, for example S-shaped so as to define a casing **43** with an internal separator **44** and the two portions **41** and **42.**

Therefore, the internal chamber **430** may be divided into two housing areas **431** and **432.**

For example, the area **431** may house the diffuser means **21** of the portion **41,** while the area **432** may house the one or more air chambers **31** of the portion **42.**

Even more particularly, the portion **41** may be in contact with the area of the body to be treated, so that the diffuser means **21** act without any interference whatsoever, while the portion **42** may be opposite to the portion **41.**

Therefore, in the examples mentioned above with reference to the cuff, to the leg and to the abdominal band, the portion **41** may respectively be in contact with the limb or abdomen, while in the case of a vertebral support mattress, the portion **41** may be in contact with the back along the vertebral region.

In this manner, given that the portion **42** may house the one or more air chambers **31,** the latter will not interfere with the magnetic fields of the diffuser means **21.**

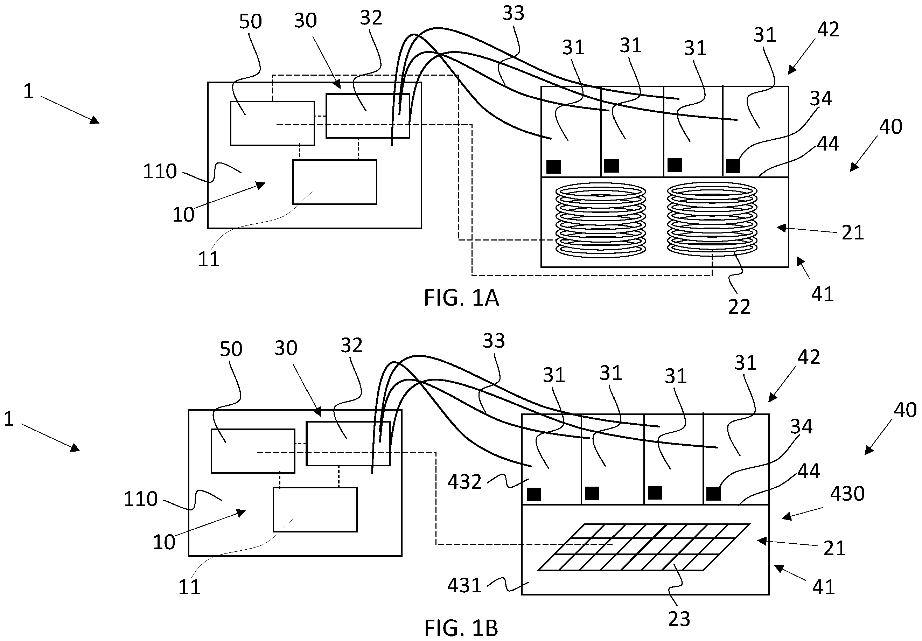

Furthermore, preferably, the diffuser means **21** may include one or more solenoids **22**, as shown in **FIG. 1A**, with variable size for generating low frequency magnetic fields and have a more precise treatment, or one or more conductive gratings **23**, as shown in **FIG. 1B**, for generating high frequency magnetic fields and have a more widespread treatment on the area of interest.

For example, such conductive gratings **23** may be in the form of copper gratings which may be integrated in the portion **41**.

Therefore, it is clear that the diffuser means **21** for magnetotherapy shall be understood as the part or parts of the device **1** which include components of the device **1** designed to generate magnetic fields, in a per se known manner.

Generally, the logic control unit **50** may alternately or simultaneously actuate the diffusers **21** and the pneumatic means **30** so as to treat the area of the body with magnetotherapy or pressotherapy or magnetotherapy and pressotherapy.

Specifically described below are all the components mentioned above.

Should the device **1** be portable, the power supply means **10** may be rechargeable and they may be in the form of a battery **11,** for example a lithium battery with a 12 Vdc voltage.

In this case, for example, the rechargeable battery **11** may be charged by connecting to the power mains only when charging and then be disconnected from the latter once fully charged, so as to contribute to making the device **1** portable.

On the other hand, the device **1** may also be used when charging the battery **11**. Suitably, the battery **11** may have a voltage comprised between 10 -14 Vdc.

Advantageously, the battery **11** may power-supply the logic control unit **50** and, by controlling the latter, the diffuser means **21** and the pneumatic means **30** in a per se known manner.

Preferably, the device **1** may include starting means **70** such as for example an *ON* / *OFF* button connected to the power supply means **10**, for example to the battery **11**.

Preferably, the starting means **70** may be positioned on the shell **100**.

Besides the starting means **70** or as a replacement thereof, the device **1** may include control means **80,** such as for example a keypad, a push-button panel or the like, connected to the power supply means **10** and to the logic control unit **50,** positioned on the shell **100.**

For example, the keypad **80** may comprise keys or it may be of the touch screen type so as to minimally affect the thickness **s** of the shell **100** and reduce the overall dimensions thereof.

Such solution will be particularly useful should the device **1** be portable.

Preferably, the logic control unit **50** may include a microcontroller **50** which may be power-supplied by the battery **11** and/or by the power mains.

In a per se known manner, the microcontroller **50** may be integrated on an electronic board configured to allow the generation of high frequency and/or low frequency magnetic fields and for actuating the pneumatic means **30.**

Preferably, the board may have a circuit board of the printed type that is it may be of the PCB type.

This will contribute to making the device **1** particularly compact and it may be advantageous should the device **1** be portable.

The control unit **50,** microcontroller **50** specifically, may comprise an internal memory with one or more therapeutic programmes which can be selected by the user depending on the therapy to be carried out, whether magnetotherapy, pressotherapy or a combination of magnetotherapy and pressotherapy.

Specifically, the device **1** may include a software programme which can be installed for example in the control unit **50** which comprises instructions adapted command the latter to alternately and selectively or simultaneously actuate the diffusers **21** and the pneumatic means **30** according to at least one predetermined therapeutic programme or which can also be set in the logic control unit **50,** as described above.

For example, the therapeutic programme may provide for the actuation of the pneumatic means **30** alone and therefore the filling, possibly differentiated, of the one or more air chambers **31** or only the activation of the diffusers **21** or even the alternative actuation, over time or simultaneous, of the pneumatic means **30** and of the diffusers **21.**

In a preferred but non-exclusive embodiment, the device **1** may include a wireless reception module **90** connected to the logic unit **50,** specifically with the microcontroller **50,** in a per se known manner.

In this case, there may be provided for a system **2** which includes the device **1** and a smartphone **SM,** as shown in **FIG. 3****.**

Preferably, the smartphone **SM** may have the computer programme mentioned above, in the form of an installed application.

In this case, the therapeutic programme may be stored in the logic unit **50** and/or it may be allocated in a remote database and/or it may be suitable to be set directly on the smartphone **SM.**

In this manner, the user may select one or more therapeutic programmes directly in the application of the smartphone **SM** so that the wireless transmission module of the latter sends such data to the module **90** of the device **1** to run the selected therapeutic programmes.

Advantageously, the pneumatic means **30** may include a compressor **32,** per se known, of the connection cables **33** between the compressor **32** and the one or more air chambers **31,** per se known, and of the pressure sensors **34** inside the latter.

Therefore, it is clear that the pneumatic means **30** shall be understood as the part or parts of the device **1** which include the components of the device **1** designed to generate compressed air or other compressed gases, in a per se known manner.

In a preferred but non-exclusive embodiment, the portion **42** may include a plurality of air chambers **31** independent from each other from a fluidic standpoint.

This means that each of them may be connected by means of a respective cable **33** to the compressor **32** and each of them may internally include a pressure sensor **34** so as to detect the internal pressure reached in each chamber **31** upon the actuation of the compressor **32.**

It is therefore clear that the logic unit **50** may control the differentiated inflation of each air chamber **31,** as known in pressotherapy, and simultaneously verify the pressure level achieved.

Advantageously, the adjustable pressure inside each chamber **31** may be between 0 and 300 mmHg.

However, it is clear that the pressure sensors **34** may also not be provided for and the inflation may be carried out without any retroactive control without departing from the scope of protection of the attached claims.

In the light of the above, the present invention attains the pre-established objects.

The invention is susceptible to numerous modifications and variants. All details can be replaced by other technically equivalent elements, and the materials can be different depending on the needs, without departing from the scope of protection of the invention defined by the attached claims.

## Claims

1. A versatile multifunction device for pressotherapy and/or magnetotherapy, which can be used by a user to treat at least one area of the body, comprising:
- at least one shell (**100**) defining at least one inner compartment (**110**) which includes:
- at least one logic control unit (**50**);
- power supply means (**10**) operatively connected to said at least one logic control unit (**50**);
- pneumatic means (**30**) operatively connected to said at least one logic control unit (**50**);
- at least one support (**40**) which can be anchored to the user's area to be treated, comprising at least one first portion (**41**) which includes diffuser means (**21**) for magnetotherapy operatively connected to said at least one logic control unit (**50**) and at least one second portion (**42**) comprising at least one air chamber (**31**) for pressotherapy fluidically connected to said pneumatic means (**30**);
wherein said at least one logic control unit (**50**) alternatively or simultaneously actuates said diffuser means (**21**) and said pneumatic means (**30**) so as to treat the at least one area of the user's body.

2. Device according to the preceding claim, wherein the device is portable, said power supply means (**10**) being rechargeable and preferably including at least one rechargeable battery (**11**).

3. Device according to the preceding claim, wherein said at least one rechargeable battery (**11**) can be charged by connecting with the power mains, the device (**1**) being suitable to be used both when charging said at least one rechargeable battery (**11**) and when the latter is electrically disconnected from the power mains.

4. Device according to the preceding claim, wherein said at least one rechargeable battery (**11**) has a voltage comprised between 10 -14 Vdc.

5. Device according to any one of the preceding claims, wherein said at least one support (**40**) includes at least one leg and/or at least one cuff and/or at least one abdominal band and/or at least one mattress for vertebral support.

6. Device according to any one of the preceding claims, wherein said at least one support (**40**) includes at least one casing (**43**) defining an internal chamber (**430**) and at least one separator (**44**), the latter dividing said internal chamber (**430**) in at least one first and one second housing area (**431, 432**) respectively for said diffuser means (**21**) of said at least one first portion (**41**) and said at least one air chamber (**31**) of said at least one second portion (**42**).

7. Device according to the preceding claim, wherein said at least one first portion (**41**) is at least partially mutually in contact with the at least one area of the user's body, said at least one second portion (**42**) remaining substantially opposite to said at least one first portion (**41**).

8. Device according to claim 6 or 7, wherein said at least one casing (**43**) is made of technical fabric or natural fibre.

9. Device according to any one of claims 6 to the preceding one, wherein said diffuser means (**21**) include at least one solenoid (**22**) and/or at least one conductive grating (**23**) inserted into said at least one first housing area (**431**).

10. Device according to one or more of the preceding claims, wherein said at least one second portion (**42**) includes a plurality of said at least one air chamber (**31**), each being mutually connected to said pneumatic means (**30**) in a fluidically independent manner, said at least one logic control unit (**50**) commanding the latter to carry out the differentiated inflation of each air chamber of said plurality of said at least one air chamber (**31**).

11. Device according to one or more of the preceding claims, wherein said at least one logic control unit (**50**) alternately or simultaneously actuates or said diffuser means (**21**) and said pneumatic means (**30**) so as to treat the area of the body with magnetotherapy or pressotherapy or magnetotherapy and pressotherapy.

12. Device according to one or more of the preceding claims, wherein said at least one logic control unit (**50**) comprises at least one microcontroller (**50**) integrated on an electronic board suitably configured to allow the generation of high frequency and/or low frequency magnetic fields and for actuating said pneumatic means (**30**).

13. Device according to the preceding claim, wherein said electronic board includes a circuit board of the printed circuit board type.

14. Device according to claim 12 or 13, wherein said at least one microcontroller (**50**) comprises an internal memory with one or more therapeutic programmes which can be selected by the user depending on the therapy to be carried out, the latter being magnetotherapy, pressotherapy or a combination of magnetotherapy and pressotherapy.

15. Computer program comprising instructions adapted to control the at least one logical control unit (**50**) of a device (**1**) according to one or more of claims 1 to the preceding one, to alternatively or simultaneously actuate said diffuser means (**21**) and said pneumatic means (**30**) according to at least one therapeutic programme that is predetermined or which can be set in the at least one control logic unit (**50**).
